# FASCICULE DE BREVET EUROPEEN

(11) **EP 1 199 993 B1**
(45) Date de publication et mention de la délivrance du brevet: **04.04.2007**
(21) Numéro de dépôt: 00949602.7
(22) Date de dépôt: 30.06.2000
(51) Int. Cl.: A61B 17/70

(54) **ELEMENT DE FIXATION ET ANCILLAIRE POUR LA STABILISATION DES VERTEBRES**
BEFESTIGUNGSVORRICHTUNG UND HILFSGERÄT ZUR STABILISIERUNG DER WIRBEL
FIXING ELEMENT AND ANCILLARY FOR STABILISING VERTEBRAE

(30) Priorité: 01.07.1999 FR 9908498
(43) Date de publication de la demande: 02.05.2002
(73) Titulaire: Spinevision S.A., 75012 Paris (FR)
(72) Inventeur: VANACKER, Gérard, F-94100 Saint-Maur (FR); ZELLER, Reinhard, 92100 Boulogne (FR)
(74) Mandataire: Breese, Pierre
(86) Numéro de dépôt international: PCT/FR2000/001872
(87) Numéro de publication internationale: WO 2001/001873

(56) Documents cités:
- EP-A- 0 346 521
- EP-A- 0 535 623
- EP-A- 0 879 579
- DE-A- 4 107 480
- US-A- 5 562 663

## Description

La présente invention concerne le domaine de l'ostéosynthèse rachidienne destinée à la chirurgie du rachis pour la correction de malformations d'ordre dégénératives ou idiopathiques, ou neuromusculaires ou tumorales, ou de traumatologie.

Il est connu dans l'art antérieur d'utiliser une instrumentation comportant des implants qui sont fixés sur l'os, par vissage ou par des crochets et des éléments de liaison qui permettent au chirurgien d'appliquer des contraintes pour le redressement ou la stabilisation du rachis. Un implant selon le préambule de la revendication 1 est connu par exemple du document DE 4107480.

À titre d'exemple, le brevet européen EP626828 décrit un tel système permettant de pratiquer l'ostéosynthèse sur la colonne vertébrale, ainsi qu'un élément de raccordement de ce système et les outils utilisés pour le monter et/ou le démonter. Ce document de l'art antérieur décrit un dispositif pour ostéosynthèse sur la colonne vertébrale, en particulier pour la stabilisation des vertèbres, comprenant :
- au moins un élément de liaison en forme de baguette,
- au moins deux moyens de fixation susceptibles d'être chacun ancrés dans une vertèbre, ces moyens présentant une tête de type en forme de fourche dont les deux branches définissent un espace de réception sensiblement en forme de U pour l'élément de liaison,
- une vis de serrage susceptible d'être vissée dans l'espace de réception pour fixer l'élément de liaison entre les deux branches de la tête de vis en forme de fourche,
- la tête dont le fond de l'espace de réception est façonné sous la forme d'une cuvette concave en correspondance avec un élément de coussinet de basculement agencé entre le fond de l'espace de réception et l'élément de liaison, élément de coussinet dont 1a surface d'appui en vis-à-vis du fond de l'espace de réception est de forme convexe complémentaire, caractérisé en ce que l'élément de coussinet de basculement est prévu avec une surface d'appui hémisphérique en correspondance avec le fond de l'espace de réception. Cet élément est prévu avec une échancrure ouverte du côté de la vis de serrage pour la réception de l'élément de liaison en forme de baguette. L'élément de coussinet de basculement est retenu contre le fond de l'espace de réception de telle sorte qu'il peut basculer aussi bien dans un plan parallèle au plan médian de l'espace de réception que dans un plan perpendiculaire.

On connaît également la demande internationale de brevet WO 91/01691 décrivant un dispositif de redressement et d'étaiement d'un rachis constitué d'implants vissés ou de crochets reliés par au moins deux tiges solidarisées entre elles par l'intermédiaire d'éléments de raccordement et de liaison. Ces tiges sont introduites longitudinalement dans des rainures perpendiculaires à la vis, prévues à cet effet dans le corps des implants ou des crochets, puis bloquées dans le fond des rainures. Une solidarisation entre des tiges est obtenue par une traverse filetée. Une déformation par rapprochement des côtés de la rainure, aménagée dans les corps des implants ou des crochets, afin d'obtenir le blocage de la tige dans le fond de la rainure, est obtenue par l'intermédiaire d'un système à vis cylindrique et à écrou à filetage conique.

On connaît également la demande internationale de brevet WO 95/14437 divulguant un implant comprenant une partie destinée à l'ancrage osseux et un corps de fixation sur une tige, comportant deux branches latérales délimitant un canal, cet implant comprenant également un bouchon fileté adapté pour pouvoir être vissé sur les parois intérieures des deux branches.

La demande internationale de brevet WO 94/10944 décrit un dispositif qui comprend un élément de connexion se trouvant entre une tige, ou autre implant longitudinal, et une vis de fixation d'os placée dans la vertèbre dégénérative. Cet élément de connexion comprend une bague dimensionnée de telle sorte que la tige puisse la traverser. La bague est pourvue de vis pour fixer la tige et s'étend radialement grâce à un bras cylindrique conçu pour être fixé à la vis de fixation de l'os et serré sur la vis. Le bras et la bague constituent une seule pièce. Ce dispositif permet d'éviter au chirurgien de déformer davantage la tige lorsque celle-ci est en présence de pédicules non alignés en laissant la totale liberté au chirurgien de choisir l'emplacement des deux axes de la vis de fixation de l'os et de la tige.

La demande de brevet européen EP 346 521 décrit un élément de fixation susceptible d'être ancré sur une vertèbre, pour l'instrumentation d'ostéosynthèse, du type présentant une tête en forme de fourche dont les deux branches définissent un espace de réception sensiblement en forme de U pour l'élément de liaison, le fond de la tête présentant une forme générale de selle de cheval.

Le problème posé par les dispositifs de l'art antérieur est celui du maintien de l'orientation décidée par le chirurgien lors de la fixation définitive et de la dissociation entre le positionnement de l'élément de liaison et la pose de l'implant osseux. Dans les dispositifs comportant une vis de serrage, le serrage de cette vis à tendance à modifier l'orientation relative des tiges de liaison et de l'élément de fixation.

Un autre problème est celui de l'ajustement du réglage des vis pendant l'étape de réalignement du rachis par rotation de la tige de correction. Cette tige de correction traverse de nombreux implants vertébraux, 10 à 15 implants par exemple et/ou des vis vertébrales. Chacun de ces implants comporte en général une vis de blocage. Cette vis de blocage doit être suffisamment serrée pour ne pas s'échapper de l'implant vertébral, mais pas trop serrée pour permettre une rotation sans friction excessive de la tige. Le réglage du degré de vissage de chacune de ces vis de blocage est une opération fastidieuse et délicate.

L'invention vise à éviter ces inconvénients en proposant un système et un implant permettant de préserver le degré de correction décidé par le chirurgien pendant les opérations de fixation définitive et de serrage des vis de blocage de l'élément de liaison. Le but est également de permettre une rotation de la tige lors de la manoeuvre de rotation de celle-ci, avec une friction minimale indépendante du degré de serrage de la vis de blocage.

A cet effet, l'invention concerne dans son acception la plus générale un dispositif pour ostéosynthèse sur la colonne vertébrale, en particulier pour la stabilisation des vertèbres, comprenant :
- au moins un élément de liaison en forme de tige, ou de plaque,
- au moins deux moyens de fixation susceptibles d'être chacun ancré dans une vertèbre, ces moyens présentant une tête en forme de fourche dont les deux branches définissent un espace de réception sensiblement en forme de U pour l'élément de liaison,
- une vis de blocage susceptible d'être vissée dans l'espace de réception pour fixer l'élément de liaison positionné entre les deux branches de la tête de vis en forme de fourche, caractérisé en ce que le fond de la tête présente une forme générale de selle de cheval, en ce que la tête présente un moyen de guidage pour une pièce de fermeture indépendante apte à être fixé sur la tête après positionnement de l'élément de liaison dans la fourche de la tête, ladite pièce de fermeture présente une forme générale de "U" dont les branches viennent coopérer avec les branches de la partie en forme de fourche en « Y » de la tête, et dont le fond comporte un taraudage pour la coopération avec la vis de blocage et en ce que le moyen de guidage prévu sur la tête est formé par un épaulement arqué sur les surfaces latérales extérieures de la partie en forme de fourche.

La forme de selle de cheval peut être qualifiée de paraboloïde hyperbolique inversée, selon un mode particulier de réalisation mais ne se limite pas à une forme engendrée par une équation du second degré correspondant à une paraboloïde hyperbolique.

Ce mode de réalisation permet de poser tout d'abord les moyens de fixation sur le rachis, puis les éléments de liaison éventuellement en même temps que la pièce de fermeture, d'ajuster ensuite la correction indépendamment de l'élément de verrouillage, et lorsque la correction optimale est obtenue, de bloquer chaque implant par les vis de blocage. Le serrage de la vis de blocage ne modifie pas l'orientation du moyen de fixation en raison du degré de liberté de mouvement de la pièce de fermeture par rapport au corps du moyen de fixation.

La pièce de fermeture présente des branches latérales déformables élastiquement, avec un épaulement arqué sur les surfaces intérieures. Les dimensions et formes de branches et des épaulements sont déterminées de façon à permettre la mise en place de la pièce de fermeture par écartement élastique des branches et l'emboîtement des épaulements. Le verrouillage définitif de la pièce de fermeture et de l'implant est réalisé par le serrage de la vis de blocage. Le moyen de guidage de la pièce de fermeture sur la tête est formé par un épaulement arqué complémentaire prévu sur les surfaces latérales extérieures de la partie en forme de fourche. Cet épaulement arqué permet une rotation de la pièce de fermeture par rapport à la tête de l'implant.

Une variante consiste à réaliser une pièce de fermeture présentant une mémoire de forme. Une telle pièce présente des bras écartés au repos, pour permettre la mise en place sur la partie en forme de fourche. Lorsqu'elle est positionnée sur la tête du crochet, une modification de la température provoque le repliement des bras dans une position d'ancrage sur la fourche.

Selon un mode de réalisation préféré, les épaulements présentent des surfaces de contact convergent sensiblement vers le taraudage destiné à recevoir la vis de blocage.

Selon une variante, la position de la pièce de fermeture est fixe par rapport à la fourche de la tête. L'épaulement ou les moyens d'encliquetage ne permettent pas, selon cette variante, de degré de liberté en rotation de la pièce de fermeture.

L'angle de convergence n'est pas très critique. Il est seulement important que les surfaces de contact soient orientées vers le fond de la pièce de fermeture. Toutefois, un angle d'une dizaine de degrés par rapport au plan transversal permettrait déjà d'obtenir un guidage satisfaisant.

Selon une variante de réalisation, la tête est prolongée par une partie inférieure en forme de crochet de maintien du rachis pour la mise en place sur un pédicule, lame de vertèbre ou apophyse transverse, ledit crochet comportant une lame élastique pour le maintien provisoire de la fixation.

L'invention se rapporte certes au système comportant l'ensemble des composants (tige de correction, implants, pièces de fermeture, vis de blocage). Elle concerne toutefois également l'élément de fixation susceptible d'être ancré sur une vertèbre, pour l'instrumentation d'ostéosynthèse. Cet élément de fixation peut être utilisé avec d'autres éléments de liaison que des tiges, par exemple un élément de liaison à section trapézoïdale ou variable, ou des lames, notamment des lames présentant des zones de liaison à section circulaire.

L'invention concerne également un ancillaire pour la mise en oeuvre d'un système pour ostéosynthèse conforme à l'invention caractérisé en ce qu'il présente deux becs venant se loger dans les échancrures prévues sur la tête de l'implant, et un organe venant exercer un effort sur la tige pour assurer son déplacement latéral et ou vertical, en vue de permettre le positionnement de la tige dans la fourche. Cet effort peut être exercé sur la tige par l'intermédiaire de la pièce de fermeture avec laquelle l'ancillaire coopère pendant la phase de mise en place de la tige dans la fourche de l'implant. La coopération peut être réalisée par vissage provisoire de l'ancillaire dans le taraudage de la pièce de fermeture.

L'invention sera mieux comprise à la lecture de la description qui suit, se rapportant aux dessins annexés où :
- la figure 1 représente une vue de coupe longitudinale d'un exemple de réalisation d'un système d'ostéosynthèse selon l'invention ;
- la figure 2 représente une vue schématique de côté du système selon l'invention ; et
- les figures 3 et 4 représentent une vue de l'implant selon deux vues de faces perpendiculaires.

Le système pour ostéosynthèse selon la présente invention comporte un élément de liaison (1), un implant (2) avec une pièce de fermeture (3) complémentaire, et une vis de blocage (13).

La tige (1) ne sera pas décrite plus en détail car elle fait partie de l'état de la technique et peut prendre diverses formes. Dans l'exemple décrit, elle est formée par une tige métallique à section circulaire.

L'implant présente une tête (5) en forme de fourche, présentant deux bras latéraux (6, 7) délimitant un espace destiné à recevoir l'élément de liaison (1).

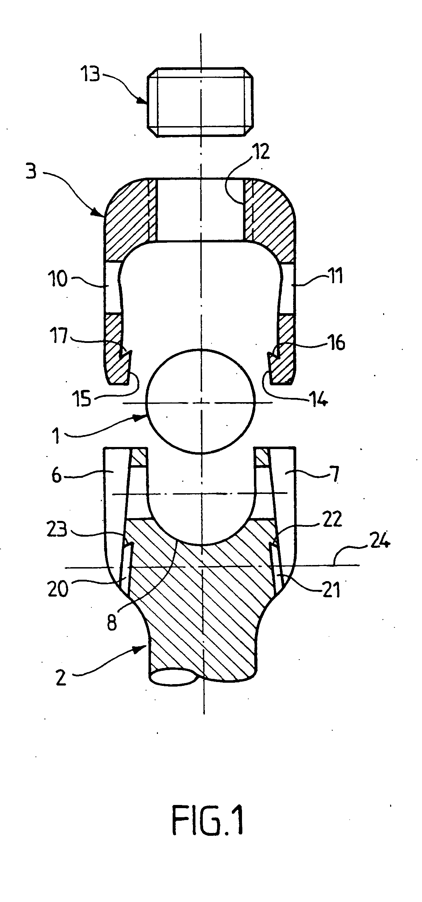

Le fond (8) de la fourche présente une forme générale de fer à cheval, avec une courbure concave dans le plan transversal correspond au plan de la figure 1, et une courbure convexe dans le plan complémentaire.

Le rayon de courbure concave correspond sensiblement au rayon extérieur de l'élément de guidage (1). Ce dernier vient ainsi en contact selon une ligne semi-périphérique. Ce contact selon une ligne et non pas selon une surface annulaire autorise un degré de liberté en pivotement, et assure en même temps un blocage plus efficace après serrage que dans le cas d'un simple contact ponctuel.

La pièce de fermeture (3) présente une forme générale de "U", avec deux bras (10, 11) et un fond présentant un taraudage (12) pour recevoir une vis de blocage.

Les bras (10, 11) présentent un écartement permettant la mise en place sur la tête. Les bras (10, 11) présentent à leur extrémité inférieure des épaulements arqués (14, 15) avec une surface supérieure (16, 17) inclinée.

Ces épaulements arqués (14, 15) viennent coopérer avec des moyens de guidage complémentaires (20, 21) prévus sur la tête (5). Ces moyens de guidages présentent également une surface de contact arqué (22, 23) inclinés, et viennent coopérer avec les surfaces de contact complémentaires (14, 15) lorsque la pièce de fermeture est mise en place sur la tête (5). Elles assurent alors un guidage permettant le pivotement de la pièce de fermeture selon un axe (24) transversal et assurent le verrouillage de la pièce de fermeture (3) sur la tête (5), et donc le blocage de la tige (1) après serrage de la vis (13).

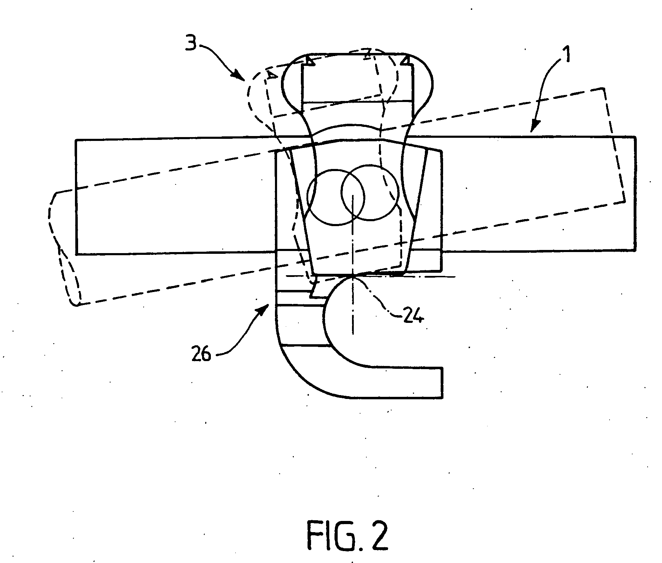

La figure 2 représente une vue de côté qui montre que la tige (1) dispose d'un degré de liberté en basculement autour d'un axe transversal (24). Ceci permet de donner une indépendance à l'implant, et de positionner l'implant au moyen du crochet (26) sur le pédicule, et indépendamment à chercher la meilleure orientation de la tige (1) sans interférence entre ces deux contraintes . La forme de fer à cheval et la mobilité de la pièce de fermeture permet d'adapter le verrouillage et d'éviter la dérotation ou le déplacement de la tige lors du serrage de la vis (13).

Le crochet (26) délimite un espace (27) en "U" pour la liaison avec la lame d'une vertèbre. Afin d'assurer un maintien temporaire, une lame élastique (28) est disposée à l'intérieur de cet espace en "U" et assure un maintien temporaire sur l'os de façon à ce que la lame du crochet ne risque pas de perturber la moelle ou autre structure.

La lame élastique (28) repousse le crochet dans une direction postérieure par rapport au patient, et évite les lésions des tissus nobles pendant la phase de correction par rotation de la tige.

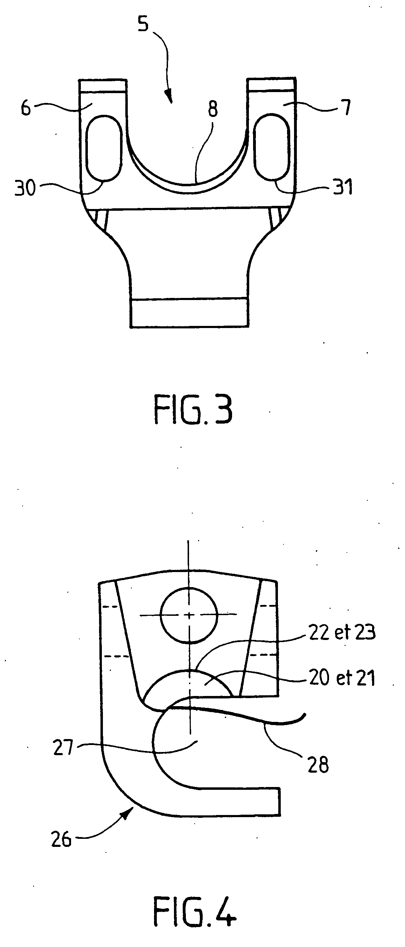

Les figures 3 et 4 représentent des vues de côté de l'implant, sans la pièce de fermeture.

L'implant présente deux échancrures (30, 31) permettant le passage d'un instrument présentant deux becs venant se loger dans les échancrures (30, 31), et un organe venant exercer un effort sur la tige pour assurer son déplacement latéral et / ou vertical, en vue de permettre le positionnement de la tige dans la fourche par l'intermédiaire de la pièce de fermeture (3). Dans ce cas, une partie de l'instrument est provisoirement solidarisée avec la pièce de fermeture à l'aide d'une vis introduite dans le taraudage (12) de la pièce de fermeture (3).

L'invention est décrite dans ce qui précède à titre d'exemple non limitatif. I1 est entendu que l'homme du métier peut réaliser diverses variantes, notamment en remplaçant le crochet par une vis pédiculaire, ou une vis vertébrale pour la pose sur la face antéro-latérale du rachis.

## Revendications

1. - Implant, du type comprenant un élément de fixation susceptible d'être ancré sur une vertèbre et présentant une tête (5) en forme de fourche dont les deux branches définissent un espace de réception sensiblement en forme de "U", destiné à recevoir un élément de liaison, et une pièce de fermeture (3) apte à être fixée sur la tête (5), ledit implant étant **caractérisé en ce que** la pièce de fermeture (3) présente une forme générale de "U" dont les branches viennent coopérer avec les branches de la partie en forme de fourche de la tête (5), et **en ce que** la tête (5) présente, sur le fond du "U", une forme générale de selle de cheval permettant le pivotement de l'élément de liaison (1) autour d'un axe transversal (24) audit implant, lorsque ledit élément de liaison est reçu dans l'espace de réception, et sur les surfaces latérales extérieures de la partie en forme de fourche, un moyen de guidage pour la pièce de fermeture (3), ledit moyen de guidage étant formé par un épaulement arqué de sorte à permettre le pivotement de la pièce de fermeture (3) selon l'axe transversal (24).

2. - Implant selon la revendication 1, **caractérisé en ce que** la pièce de fermeture (3) présente un épaulement complémentaire, les dimensions et formes d'épaulements étant déterminées de façon à permettre la mise en place de la pièce de fermeture (3).

3. - Implant selon la revendication 2, **caractérisé en ce que** les épaulements sont configurés pour permettre la mise en place de la pièce de fermeture (3) par écartement élastique ou mémoire de forme des branches.

4. - Implant selon la revendication 2 ou la revendication 3, **caractérisé en ce que** les épaulements prévus sur les surfaces latérales de la partie en forme de fourche de la tête (5) sont en forme d'arc de cercle, permettant un degré de liberté unique de la pièce de fermeture (3) par rapport à l'élément de fixation sur le rachis.

5. - Implant selon l'une quelconque des revendications 1 à 4, **caractérisé en ce qu'**il est prolongé par une partie inférieure en forme de crochet pour la mise en place sur la vertèbre, ledit crochet comportant une lame élastique (28) pour le maintien provisoire de la fixation.

6. - Système pour ostéosynthèse sur la colonne vertébrale, en particulier pour la stabilisation des vertèbres, comprenant au moins deux implants selon l'une quelconque des revendications 1 à 5 susceptibles d'être chacun ancrés sur une vertèbre, et au moins un élément de liaison (1) en forme de tige, disposé dans l'espace de réception de la tête (5) desdits implants, l'élément de liaison (1) étant fixé dans l'espace de réception de la tête (5) de chaque implant par une vis de blocage (13).

7. - Système pour ostéosynthèse sur la colonne vertébrale selon la revendication 6, **caractérisé en ce que** la pièce de fermeture (3) présente un épaulement complémentaire des moyens de guidage, les dimensions et formes d'épaulements étant déterminées de façon à permettre la mise en place de la pièce de fermeture (3) par écartement élastique ou mémoire de forme des branches et l'ancrage par venue en contact des surfaces transversales des épaulements lors du serrage de la vis de blocage (13).

8. - Système pour ostéosynthèse sur la colonne vertébrale selon la revendication 7, **caractérisé en ce que** les épaulements prévus sur les surfaces latérales de la partie en forme de fourche de la tête (5) sont en forme d'arc de cercle.

9. - Système pour ostéosynthèse sur la colonne vertébrale selon l'une quelconque des revendications 6 à 8, **caractérisé en ce que** le fond du "U" de la pièce de fermeture (3) comporte un taraudage (12) pour la coopération avec la vis de blocage (13).

10. - Système pour ostéosynthèse sur la colonne vertébrale selon la revendication 9, **caractérisé en ce que** les épaulements présentent des surfaces de contact convergeant sensiblement vers le taraudage (12) destiné à recevoir la vis de blocage (13).

11. - Système pour ostéosynthèse sur la colonne vertébrale selon l'une au moins des revendications 6 à 10, **caractérisé en ce que** la tête (5) est prolongée par une partie inférieure en forme de crochet pour la mise en place sur un pédicule ou autre, ledit crochet comportant une lame élastique pour le maintien provisoire.

12. - Ancillaire pour la mise en oeuvre d'un système pour ostéosynthèse conforme à l'une quelconque des revendications 6 à 11, **caractérisé en ce que** l'ancillaire présente deux becs venant se loger dans des échancrures (30, 31), prévues sur la tête (5) de l'implant, et un organe venant exercer un effort sur la pièce de fermeture (3) pour assurer le déplacement latéral et ou vertical de l'élément de liaison, en vue de permettre son positionnement dans l'espace de réception de la fourche.

13. - Ancillaire selon la revendication 12, **caractérisé en ce qu'**il présente une zone de liaison avec la pièce de fermeture (3) par l'intermédiaire d'une vis provisoire coopérant avec le taraudage (12).

## Claims

1. Implant, of the type comprising a fastener that can be anchored to a vertebra and having a head (5) in the shape of a fork in which the two branches define a substantially U-shaped reception space, intended for receiving a link element, and a closure part (3) that can be fixed to the head (5), said implant being **characterised in that** the closure part (3) is generally U-shaped, whose the branches cooperate with the branches of the fork-shaped part of the head (5), and **in that** the head (5) has, at the bottom of the "U", the general shape of a horse saddle allowing the link element (1) to pivot about a transverse axis (24) of said implant, when said link element is housed in the reception space, and means of guiding the closure part (3) on the outer side surfaces of the fork-shaped part, said guiding means being formed by an arched shoulder such as to allow the closure part (3) to pivot about the transverse axis (24).

2. Implant according to claim 1, **characterised in that** the closure part (3) has a complementary shoulder, the dimensions and shapes of the shoulders being determined so as to allow the installation of the closure part (3).

3. Implant according to claim 2, **characterised in that** the shoulders are configured so as to allow the installation of the closure part (3) by elastic spacing or shape memory of the branches.

4. Implant according to claim 2 or claim 3, **characterised in that** the shoulders provided on the side surfaces of the fork-shaped part of the head (5) are in the shape of an arc of a circle, allowing a unique degree of freedom of the closure part (3) with regard to the element for attachment to the vertebral column.

5. Implant according to any one of the claims from 1 to 4, **characterised in that** it is extended by a lower hook-shaped part for installation on the vertebra, said hook comprising a spring plate (28) for provisional holding of the fastener.

6. System for spinal osteosynthesis, in particular for stabilising vertebrae, comprising at least two implants according to any one of the claims from 1 to 5, each of which is capable of being anchored to a vertebra, and at least one link element (1) in the form of a rod, disposed in the reception space of the head (5) of said implants, the link element (1) being fixed in the reception space of the head (5) of each implant by a lock screw (13).

7. System for spinal osteosynthesis according to claim 6, **characterised in that** the closure part (3) has a shoulder that complements the guiding means, the dimensions and shapes of the shoulders being determined such as to allow the installation of the closure part (3) by elastic separation or shape memory of the branches and anchoring by coming into contact with the transverse surfaces of the shoulders when tightening the lock screw (13).

8. System for spinal osteosynthesis according to claim 7, **characterised in that** the shoulders provided on the side surfaces of the fork-shaped part of the head (5) are in the shape of an arc of a circle.

9. System for spinal osteosynthesis according to any one of the claims from 6 to 8, **characterised in that** the bottom of the "U" of the closure part (3) comprises a threaded hole (12) for cooperation with the lock screw (13).

10. System for spinal osteosynthesis according to claim 9, **characterised in that** the shoulders have contact surfaces that converge substantially towards the threaded hole (12) intended for receiving the lock screw (13).

11. System for spinal osteosynthesis according to at least one of the claims from 6 to 10, **characterised in that** the head (5) is extended by a hook-shaped bottom part for installation on one pedicle or another, said hook comprising a spring plate for provisional holding.

12. Ancillary for implanting a system for osteosynthesis according to any one of the claims from 6 to 11, **characterised in that** the ancillary has two lips that become lodged in notches (30, 31) made in the head (5) of the implant, and an element that exerts a force on the closure part (3) in order to ensure the lateral and/or vertical movement of the link element, with a view to allowing it to be positioned in the reception space of the fork.

13. Ancillary according to claim 12, **characterised in that** it has an area for linking with the closure part (3) by means of a provisional screw cooperating with the threaded hole (12).

## Patentansprüche

1. - Implantat von der Art mit einem Befestigungselement, das auf einem Wirbel verankert werden kann und einen Kopf (5) in Form einer Gabel aufweist, deren zwei Zinken einen Aufnahmeraum beschreiben, der in etwa "U"-förmig und dazu bestimmt ist, ein Verbindungselement aufzunehmen, und ein Verschlußteil (3), das auf dem Kopf (5) befestigt werden kann, wobei das besagte Implantat **dadurch gekennzeichnet ist, daß** das Verschlußteil (3) eine allgemeine "U"-Form aufweist, deren Zinken mit den Zinken des gabelförmigen Teils des Kopfes (5) zusammenwirken, und **dadurch**, daß der Kopf (5) auf dem Boden des "U" eine allgemeine Form eines Pferdesattels aufweist, die das Schwenken des Verbindungselements (1) um eine Querachse (24) des besagten Implantats zuläßt, wenn das besagte Verbindungselement im Aufnahmeraum aufgenommen wird, und auf den äußeren Seitenflächen des gabelförmigen Teils, ein Führungsmittel für das Verschlußteil (3), wobei das besagte Führungsmittel durch eine so gebogene Schulter gebildet wird, daß sie das Schwenken des Verschlußteils (3) nach der Querachse (24) zuläßt.

2. - Implantat nach Anspruch 1, **dadurch gekennzeichnet, daß** das Verschlußteil (3) eine zusätzliche Schulter aufweist, wobei die Abmessungen und Formen der Schultern so bestimmt sind, daß sie das Anbringen des Verschlußteils (3) erlauben.

3. - Implantat nach Anspruch 2, **dadurch gekennzeichnet, daß** die Schultern so konfiguriert sind, daß sie das Anbringen des Verschlußteils (3) durch elastisches Dehnen oder Formgedächtnis der Zinken erlauben.

4. - Implantat nach Anspruch 2 oder Anspruch 3, **dadurch gekennzeichnet, daß** die auf den seitlichen Flächen des gabelförmigen Teils des Kopfes (5) vorgesehenen Schultern die Form eines Kreisbogens haben und dabei einen einzigartigen Grad Freiheit des Verschlußteils (3) im Verhältnis zum Befestigungsteil auf dem Rückgrat zulassen.

5. - Implantat nach einem beliebigen der vorstehenden Ansprüche 1 bis 4, **dadurch gekennzeichnet, daß** es durch einen unteren Teil in Hakenform für das Einsetzen auf dem Wirbel verlängert wird, wobei der besagte Haken ein elastisches Plättchen (28) für die provisorische Halterung der Befestigung umfaßt.

6. - System für Osteosynthese an der Wirbelsäule, insbesondere für die Stabilisierung der Wirbel, das mindestens zwei Implantate nach einem beliebigen der vorstehenden Ansprüche 1 bis 5 umfaßt, die jeweils auf einem Wirbel verankert werden können, und mindestens ein Verbindungselement (1) in Stabform, das im Aufnahmeraum des Kopfes (5) der besagten Implantate angeordnet ist, wobei das Verbindungselement (1) im Aufnahmeraum des Kopfes (5) jedes Implantats durch eine Sicherungsschraube (13) befestigt wird.

7. - System für Osteosynthese an der Wirbelsäule nach Anspruch 6, **dadurch gekennzeichnet, daß** das Verschlußteil (3) eine zusätzliche Schulter der Führungsmittel aufweist, wobei die Abmessungen und Formen der Schultern so bestimmt sind, daß sie das Anbringen des Verschlußteils (3) durch elastisches Dehnen oder Formgedächtnis der Zinken und die Verankerung durch Herstellen des Kontakts der Querflächen der Schultern bei erlauben anziehen der Sicherrungschraube (13).

8. - System für Osteosynthese an der Wirbelsäule nach Anspruch 7, **dadurch gekennzeichnet, daß** die auf den seitlichen Flächen des gabelförmigen Teils des Kopfes (5) vorgesehenen Schultern die Form eines Kreisbogens haben.

9. - System für Osteosynthese an der Wirbelsäule nach einem beliebigen der vorstehenden Ansprüche 6 bis 8, **dadurch gekennzeichnet, daß** der Boden des "U" des Verschlußteils (3) ein Innengewinde (12) für das Zusammenwirken mit der Sicherungsschraube (13) umfaßt.

10. - System für Osteosynthese an der Wirbelsäule nach Anspruch 9, **dadurch gekennzeichnet, daß** die Schultern Kontaktflächen aufweisen, die in etwa zum Innengewinde (12) konvergieren, das für die Aufnahme der Sicherungsschraube (13) bestimmt ist.

11. - System für Osteosynthese an der Wirbelsäule nach mindestens einem der vorstehenden Ansprüche 6 bis 10, **dadurch gekennzeichnet, daß** der Kopf (5) durch einen unteren Teil in Hakenform für das Einsetzen auf einer Wirbelbogenwurzel oder einer anderen (Stelle) verlängert wird, wobei der besagte Haken ein elastisches Plättchen für die provisorische Halterung umfaßt.

12. - Hilfsmittel für den Einsatz eines Systems für die Osteosynthese an der Wirbelsäule nach einem beliebigen der vorstehenden Ansprüche 6 bis 11, **dadurch gekennzeichnet, daß** das Hilfsmittel zwei Flügel aufweist, die in die Ausschnitte (30, 31) eintreten, die auf dem Kopf (5) des Implantats vorgesehen sind, und ein Organ, das eine Kraft auf das Verschlußteil (3) ausübt, um die seitliche oder senkrechte Verschiebung des Verbindungselements zu gewährleisten, damit seine Positionierung im Aufnahmeraum der Gabel möglich wird.

13. - Hilfsmittel nach Anspruch 12, **dadurch gekennzeichnet, daß** es eine Verbindungszone mit dem Verschlußteil (3) vermittels einer provisorischen Schraube aufweist, die mit dem Innengewinde (12) zusammenwirkt.
